# EUROPÄISCHE PATENTSCHRIFT

(11) **EP 0 652 931 B1**
(45) Veröffentlichungstag und Bekanntmachung des Hinweises auf die Patenterteilung: **05.06.1996**
(21) Anmeldenummer: 93915955.4
(22) Anmeldetag: 19.07.1993
(51) Int. Cl.: C11D 1/62, A61K 7/075, A61K 7/08, A61K 7/50

(54) **SCHÄUMENDE DETERGENSGEMISCHE**
FOAMING DETERGENT MIXTURES
MELANGES DETERGENTS MOUSSANTS

(30) Priorität: 27.07.1992 DE 4224714
(43) Veröffentlichungstag der Anmeldung: 17.05.1995
(73) Patentinhaber: Henkel Kommanditgesellschaft auf Aktien, 40191 Düsseldorf (DE)
(72) Erfinder: BIGORRA LLOSAS, Joaquim, E-08203 Sabadell (ES); PRAT QUERALT, Esther, E-08328 Alella (ES); PONSATI OBIOLS, Oriol, E-08025 Barcelona (ES)
(86) Internationale Anmeldenummer: EP9301903
(87) Internationale Veröffentlichungsnummer: WO9402575

(56) Entgegenhaltungen:
- EP-A- 0 259 678
- EP-A- 0 295 385
- WO-A-93/10748

## Beschreibung

### Gebiet der Erfindung

Die Erfindung betrifft schäumende Detergensgemische enthaltend quaternierte Fettsäurealkanolaminester und weitere anionische, nichtionische und/oder amphotere bzw. zwitterionische Tenside, Mittel, die diese Gemische enthalten sowie die Verwendung der Gemische zur Herstellung von Wasch-, Spül- und Reinigungsmittel und Mitteln zur Haar- und Körperpflege.

### Stand der Technik

Zur Herstellung von oberflächenaktiven Mitteln, wie beispielsweise Wasch-, Spül- und Reinigungsmitteln, Haarshampoos oder Schaumbädern, werden üblicherweise anionische, nichtionische und/oder amphotere bzw. zwitterionische Tenside benötigt, die für das Schaum-, Netz- und Reinigungsvermögen dieser Produkte verantwortlich sind. Der Zusatz von kationischen Tensiden zu diesen Mitteln ist in vielen Fällen erwünscht, da von diesen oberflächenaktiven Substanzen eine avivierende, schaumstabilisierende und gegebenenfalls auch mikrobizide Wirkung ausgeht. Die Realisierung scheitert jedoch in der Regel daran, daß die Kationtenside mit in den Mitteln enthaltenden anionischen Gruppen anderer Bestandteile unlösliche Komplexe bilden, die ausfallen und beispielsweise zu Faserinkrustationen führen können, wie dies für die Kombination Alkylbenzolsulfonat/QAV bekannt ist [**J.Am.Oil.Chem.Soc. 65, 1977 (1988)**].

Die Aufgabe der Erfindung bestand somit darin, neue Detergensgemische zu entwickeln, die frei von den geschilderten Nachteilen sind.

### Beschreibung der Erfindung

Gegenstand der Erfindung sind schäumende Detergensgemische enthaltend
a) quaternierte Fettsäurealkanolaminester der Formel (I), 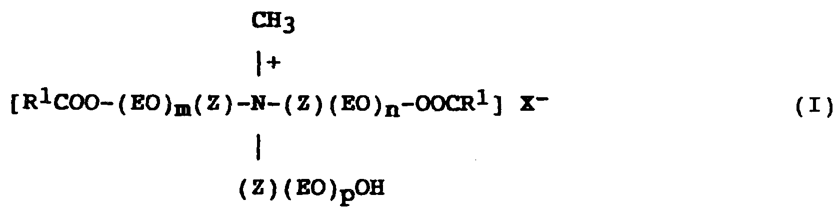 in der
   - R¹CO: für einen aliphatischen Acylrest mit 6 bis 22 Kohlenstoffatomen und 0 oder 1 Doppelbindung,
   - Z: für eine Ethylen- oder Isopropylengruppe,
   - EO: für eine CH₂CH₂O-Einheit,
   - (m+n+p): für Zahlen von 1 bis 10 und
   - X: für ein Halogenid, Methosulfat- oder Alkylphosphatanion steht,
   und
b) weitere anionische, nichtionische und/oder amphotere bzw. zwitterionische Tenside.

Überraschenderweise wurde gefunden, daß die erfindungsgemäßen Detergensgemische über ein ausgeprägtes Schaumvermögen, gute Netzeigenschaften, ausgezeichnete Wasch- und Reinigungsleistung sowie vorteilhafte ökotoxikologische und dermatologische Eigenschaften verfügen. Insbesondere wurde gefunden, daß quaternierte Fettsäurealkanolaminester der Formel (I) mit Aniontensiden bzw. anionische Gruppen enthaltenden Tensiden ausgezeichnet verträglich sind, so daß keine Bildung unlöslicher Salze bzw. Komplexe eintritt.

**Quaternierte Fettsäurealkanolaminester,** sogenannte "Esterquats", stellen bekannte Verbindungen dar, die nach den einschlägigen Methoden der präparativen organischen Chemie erhalten werden können. Ein Verfahren zu ihrer Herstellung geht von Fettsäuren und ethoxylierten Trialkanolaminen aus, die in Gegenwart von sauren Katalysatoren und einem Reduktionsmittel unter Durchleiten von Luftsauerstoff verestert und anschließend mit Dimethylsulfat quaterniert werden [**WO 91/01295,** Pulcra]. Bei den resultierenden Produkten handelt es sich um technische Mischungen, die im wesentlichen Diester und daneben Mono- und Triester enthalten.

Typische Beispiele stellen Fettsäurealkanolaminester auf Basis von Fettsäuren mit 6 bis 22, insbesondere 12 bis 18 Kohlenstoffatomen und 0 oder 1 Doppelbindung mit Anlagerungsprodukten von durchschnittlich 1 bis 10, vorzugsweise 4 bis 8 Mol Ethylenoxid an Triethanolamin oder Triisopropanolamin dar, die anschließend mit Methylhalogeniden, Dimethylsulfat oder Methylalkylphosphaten quaterniert werden. Als besonders vorteilhaft hat sich der Einsatz von Difettsäurealkanolaminestern auf Basis von Palmitinsäure, Stearinsäure, Elaidinsäure sowie technischer Talgfettsäure und Anlagerungsprodukten von durchschnittlich 4 bis 8 Mol EO an Triethanolamin erwiesen, die mit Methylchlorid oder Dimethylsulfat qua-ter-niert wurden.

Die erfindungsgemäßen schäumenden Detergensgemische werden erhalten, indem man die quaternierten Fettsäurealkanolaminester mit weiteren anionischen, nichtionischen und/oder amphoteren bzw. zwitterionischen Tensiden kombiniert, die im folgenden exemplarisch genannt werden:

### a) Anionische Tenside

Alkylbenzolsulfonate, Alkansulfonate, Olefinsulfonate, Alkylethersulfonate, Glycerinethersulfonate, α-Methylestersulfonate, Sulfofettsäuren, Alkylsulfate, Fettalkoholethersulfate, Glycerinethersulfate, Hydroxymischethersulfate, Monoglycerid(ether)sulfate, Fettsäureamid(ether)sulfate, Sulfosuccinate, Sulfosuccinamate, Sulfotriglyceride, Ethercarbonsäuren, Alkyloligoglucosidsulfate, Alkyl(ether)phosphate, Eiweißfettsäurekondensate.

### b) Nichtionische Tenside

Fettalkoholpolyglycolether, Alkylphenolpolyglycolether, Fettaminpolyglycolether, Fettsäureamidpolyglycolether, Fettsäurepolyglycolester, alkoxylierte Triglyceride, Alkyloligoglykoside, Zuckerester, Sorbitanester, Polysorbate, Polyolfettsäureester, Aminoxide, Fettsäurealkanolamide, Alkyllactame, Fettsäure-N-alkylglucamide.

### c) Amphotere bzw. zwitterionische Tenside

### Alkylamidobetaine, Aminopropionate, Aminoglycinate, Imidazoliniumbetaine, Sulfobetaine.

Die quaternierten Fettsäurealkanolaminester können in den erfindungsgemäßen Detergensgemischen in Mengen von 0,1 bis 90, vorzugsweise 5 bis 80 und insbesondere 15 bis 50 Gew.-% - jeweils bezogen auf den Feststoffgehalt der Gemische - enthalten sein.

Typische Beispiele für schäumende Detergensgemische mit besonders vorteilhaften Eigenschaften sind im folgenden genannt:
- 1 Gew.-% eines quaternierten Distearinsäure-triethanolamin-4EO-esters, quaterniert mit Dimethylsulfat im Gemisch mit 40 Gew.-% eines C_{12/14}-Kokosfettalkohol-2EO-ethersulfat-Na-Salzes (Wasser ad 100 Gew.-%);
- 5 Gew.-% eines quaternierten Ditalgfettsäure-triethanolamin-6EO-esters, quaterniert mit Dimethylsulfat im Gemisch mit 40 Gew.-% eines C_{12/14}-Kokosalkyloligoglucosids (Wasser ad 100 Gew.-%);
- 10 Gew.-% eines quaternierten Dielaidinsäuretriethanolamin-8EO-esters, quaterniert mit Methylchlorid im Gemisch mit 25 Gew.-% eines Imidazoliniumbetains (Wasser ad 100 Gew.-%).

Zur Herstellung der erfindungsgemäßen Detergensgemische ist es ausreichend, die Komponenten, gegebenenfalls unter Erwärmung auf 30 bis 40°C, zu vermischen und - falls erforderlich - zu homogenisieren. Dabei ist es sowohl möglich, von Konzentraten auszugehen, die mit Wasser auf eine Anwendungskonzentration von 1 bis 50, vorzugsweise 15 bis 30 Gew.-% verdünnt werden oder gleich verdünnte, wäßrige Ausgangsstoffe einzusetzen. In jedem Fall handelt es sich um einen rein mechanischen Vorgang; eine chemische Reaktion findet nicht statt.

Im folgenden werden weitere Gegenstände beschrieben, auf die sich die Erfindung ebenfalls erstreckt:
o **Pulverförmige Universalwaschmittel,** enthaltend 10 bis 30 Gew.-% - bezogen auf das Mittel - einer Mischung von quaternierten Fettsäureestern der Formel (I), anionischen, nichtionischen und/oder amphoteren bzw. zwitterionischen Tensiden sowie übliche Hilfs- und Zusatzstoffe.
o **Flüssige Universalwaschmittel,** enthaltend 10 bis 70 Gew.-% - bezogen auf das Mittel - einer Mischung von quaternierten Fettsäurealkanolaminestern der Formel (I), anionischen, nichtionischen und/oder amphoteren bzw. zwitterionischen Tensiden sowie übliche Hilfs- und Zusatzstoffe.
o **Flüssige Feinwaschmittel,** enthaltend 10 bis 50 Gew.-% - bezogen auf das Mittel - einer Mischung von quaternierten Fettsäurealkanolaminestern der Formel **(I)**, anionischen, nichtionischen und/oder amphoteren bzw. zwitterionischen Tensiden sowie übliche Hilfs- und Zusatzstoffe.
o **Handgeschirrspülmittel,** enthaltend 10 bis 50 Gew.-% - bezogen auf das Mittel - einer Mischung von quaternierten Fettsäurealkanolaminestern der Formel **(I)**, anionischen, nichtionischen und/oder amphoteren bzw. zwitterionischen Tensiden sowie übliche Hilfs- und Zusatzstoffe.
o **Flüssige Reinigungs- und Desinfektionsmittel,** enthaltend 10 bis 30 Gew.-% - bezogen auf das Mittel - einer Mischung von quaternierten Fettsäurealkanolaminestern der Formel **(I)**, anionischen, nichtionischen und/oder amphoteren bzw. zwitterionischen Tensiden sowie übliche Hilfs- und Zusatzstoffe.
o **Haarshampoos,** enthaltend 10 bis 30 Gew.-% - bezogen auf das Mittel - einer Mischung von quaternierten Fettsäurealkanolaminestern der Formel **(I)**, anionischen, nichtionischen und/oder amphoteren bzw. zwitterionischen Tensiden sowie übliche Hilfs- und Zusatzstoffe.
o **Haarspülungen,** enthaltend 10 bis 30 Gew.-% - bezogen auf das Mittel - einer Mischung von quaternierten Fettsäurealkanolaminestern der Formel **(I)**, anionischen, nichtionischen und/ oder amphoteren bzw. zwitterionischen Tensiden sowie übliche Hilfs- und Zusatzstoffe.
o **Schaumbäder,** enthaltend 10 bis 30 Gew.-% - bezogen auf das Mittel - einer Mischung von quaternierten Fettsäurealkanolaminestern der Formel **(I)**, anionischen, nichtionischen und/ oder amphoteren bzw. zwitterionischen Tensiden übliche Hilfs- und Zusatzstoffe.

**Wasch- und Reinigungsmittel** auf Basis der erfindungsgemäßen Detergensgemische können als Hilfs- und Zusatzstoffe beispielsweise Builder, Salze, Bleichmittel, Bleichaktivatoren, optische Aufheller, Vergrauungsinhibitoren, Lösungsvermittler und Enzyme enthalten.

Übliche **Builder** sind Natriumaluminiumsilicate (Zeolithe), Phosphate, Phosphonate, Ethylendieamintetraessigsäure, Nitrilotriacetat, Citronensäure und/oder Polycarboxylate. Als Salze bzw. Stellmittel kommen beispielsweise Natriumsulfat, Natriumcarbonat oder Natriumsilicat (Wasserglas) in Betracht. Als typische Einzelbeispiele für weitere Zusatzstoffe sind Natriumborat, Stärke, Saccharose, Polydextrose, TAED, Stilbenverbindungen, Methylcellulose, Toluolsulfonat, Cumolsulfonat, langkettige Seifen, Silicone, Mischether, Lipasen und Proteasen zu nennen.

**Haarshampoos, Haarlotionen** oder **Schaumbäder** auf Basis der erfindungsgemäßen Detergensgemische können als Hilfs- und Zustzstoffe beispielsweise Emulgatoren, Ölkomponenten, Fette und Wachse, Verdickungsmittel, Überfettungsmittel, biogene Wirkstoffe, Filmbildner, Duftstoffe, Farbstoffe, Perlglanzmittel, Konservierungsmittel und pH-Regulatoren enthalten.

Übliche **Ölkomponenten** sind Substanzen wie Paraffinöl, Pflanzenöle, Fettsäureester, Squalan und 2-Octyldodecanol, während als **Fette und Wachse** beispielsweise Walrat, Bienenwachs, Montanwachs, Paraffin und Cetylstearylalkohol Verwendung finden. Als **Überfettungsmittel** können Substanzen wie beispielsweise polyethoxylierte Lanolinderivate, Lecithinderivate und Fettsäurealkanolamide verwendet werden, wobei die letzteren gleichzeitig als Schaumstabilisatoren dienen. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate, Polyvinylalkohol und Polyvinylpyrrolidon sowie Elektrolyte wie Kochsalz und Ammoniumchlorid. Unter **biogenen Wirkstoffen** sind beispielsweise Pflanzenextrakte, Eiweißhydrolysate und Vitaminkomplexe zu verstehen. Gebräuchliche Filmbildner sind beispielsweise Polyvinylpyrrolidon, Vinylpyrrolidon-Vinylacetat-Copolymerisate, Polymere der Acrylsäurereihe, quaternäre Cellulose- Derivate und ähnliche Verbindungen. Als **Konservierungsmittel** eignen sich beispielsweise Formaldehydlösung, p-Hydroxybenzoat oder Sorbinsäure. Als **Perlglanzmittel** kommen beispielsweise Glycoldistearinsäureester wie Ethylenglycoldistearat, aber auch Fettsäuremonoglycolester in Betracht. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, veröffentlicht im Verlag Chemie, Weinheim, 1984,** zusammengestellt sind. Diese Farbstoffe werden üblicherweise in Konzentrationen von 0,001 bis 0,1 Gew.-%, bezogen auf die gesamte Mischung, eingesetzt.

### Gewerbliche Anwendbarkeit

Die erfindungsgemäßen Detergensgemische zeichnen sich durch ausgezeichnete Wasch- und Reinigungsleistung, Haar- und Textilavivage, antistatische Ausrüstung von Haaren und Fasern und Kämmbarkeitsverbesserung sowie hohe ökotoxikologische Verträglichkeit aus.

Ein weiterer Gegenstand der Erfindung betrifft daher ihre Verwendung zur Herstellung von Wasch-, Spül- und Reinigungsmitteln sowie Produkten der Haar- und Körperpflege, in denen sie in Mengen von 1 bis 50, vorzugsweise 10 bis 30 Gew.-% - bezogen auf die Mittel - enthalten sein können.

Die folgenden Beispiele sollen den Gegenstand der Erfindung näher erläutern, ohne ihn darauf einzuschränken.

### Beispiele

### I. Eingesetzte Tenside

A) Quaternierte Fettsäurealkanolaminester gemäß Formel (I)¹⁾

| | R¹CO | Z | (m+n+p) | X⁻ |
|---|---|---|---|---|
| A1 | C₁₇H₃₅CO | CH₂CH₂ | 4 | CH₃SO₄⁻ |
| A2 | C₁₇H₃₅CO | CH₂CH₂ | 6 | CH₃SO₄⁻ |
| A3 | C₁₇H₃₅CO | CH₂CH₂ | 8 | CH₃SO₄⁻ |

B) C_{12/14}-Kokosfettalkohol-2 Mol EO-sulfat-Na/Mg-Salz²⁾ Texapon^{(R)} NSO
C) C_{12/14}-Kokosfettalkoholdiethanolamid²⁾ Comperlan^{(R)} COD
D) C_{12/14}-Kokosfettsäureamidobetain²⁾ Dehyton^{(R)} K
¹⁾ Verkaufsprodukte der Fa.Pulcra S.A., Barcelona/Spanien
²⁾ Verkaufsprodukte der Fa.Henkel KGaA, Düsseldorf/FRG

### II. Anwendungstechnische Ergebnisse - Verdickbarkeit

Die verdickende Wirkung von Natriumchlorid auf Mischungen enthaltend Fettalkoholethersulfat, Fettsäurealkanolamid und gegebenenfalls Esterquats wurde in einem Brookfield-RVT-Viskosimeter (Spindel 2-7, 10 Upm) bei 20°C gemessen. Die Ergebnisse sind in **Tab.1 zusammengefaßt.**

**Tab.1:**

| Viskosität bei Zusatz von x % Natriumchlorid prozentangaben als Gew.-% | | | | | | | | | | | |
|---|---|---|---|---|---|---|---|---|---|---|---|
| Bsp | Rezeptur* | | | | | | Viskosität (1000 cps) | | | | |
| | A1 % | A2 % | A3 % | B % | C % | D % | 2% | 3% | 4% | 5% | 6% |
| 1 | 1 | - | - | 37 | 2 | - | 1 | 15 | 33 | 6 | 1 |
| 2 | - | 1 | - | 37 | 2 | - | 2 | 11 | 16 | 4 | 1 |
| 3 | - | - | 1 | 37 | 2 | - | 2 | 11 | 16 | 14 | 4 |
| 4 | 1 | - | 1 | 37 | - | 8 | 5 | 20 | 29 | 13 | 1 |
| 5 | - | 1 | - | 37 | - | 8 | 5 | 27 | 32 | 18 | 2 |
| 6 | - | - | 1 | 37 | - | 8 | 5 | 27 | 32 | 19 | 2 |
| V1 | - | - | - | 37 | 2 | - | 1 | 5 | 14 | 20 | 17 |
| V2 | - | - | - | 37 | - | 8 | 1 | 18 | 27 | 38 | 33 |

| | | | | | | | | | | | |
|---|---|---|---|---|---|---|---|---|---|---|---|
| *) Wasser ad 100 Gew.-% | | | | | | | | | | | |

### Lagerverhalten

Die verschiedenen Rezepturen wurden bei 5, 20 und 40°C gelaert und die Viskosität nach 15 bzw. 30 Tagen beurteilt. Die Ergebnisse sind in Tab.2 zusammengefaßt:

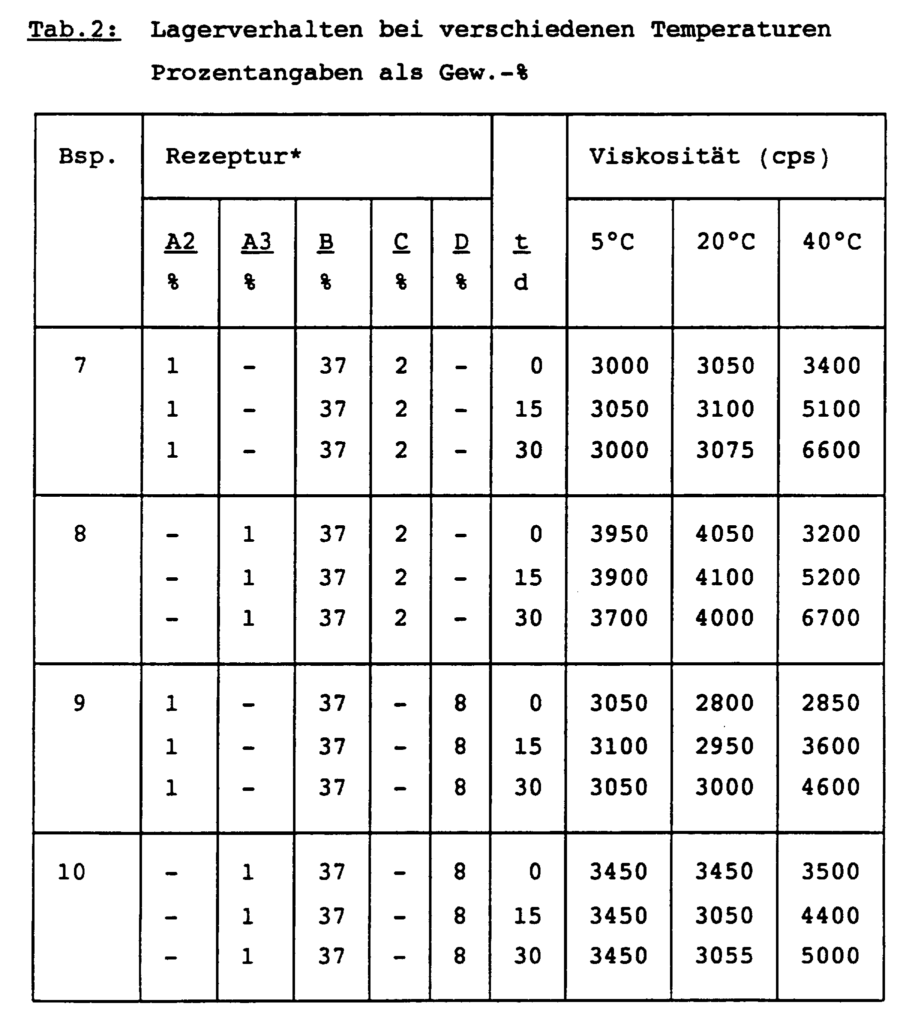

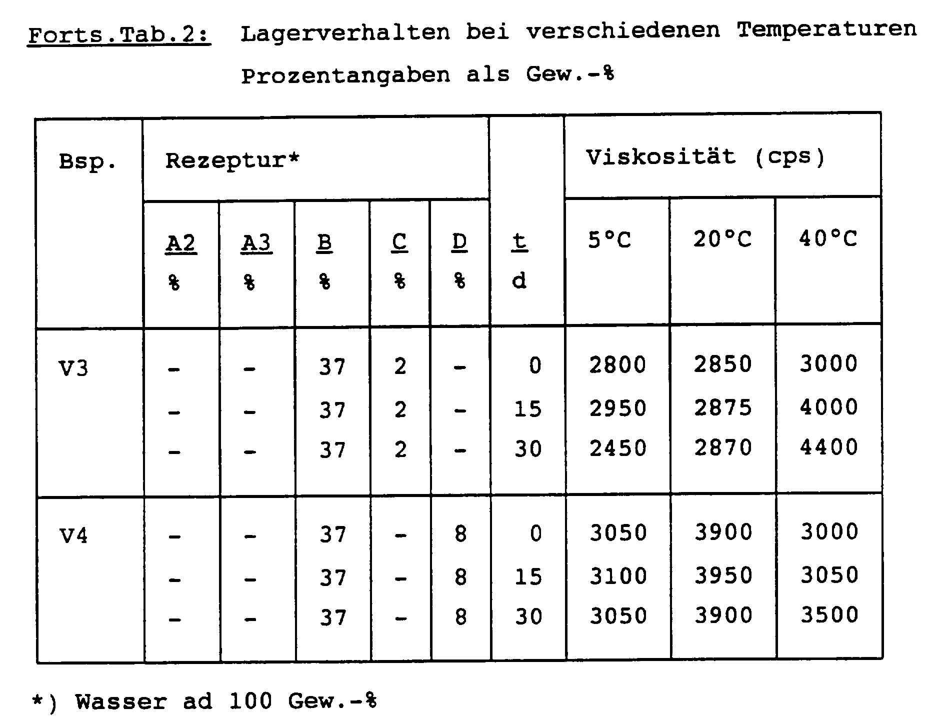

### Schaumwermögen

Das Schaumvermögen der 0,5 gew.-%igen Rezepturen wurde in einer Schlagschaumapparatur nach ROSS-MILES in Wasser einer Härte von 16°d bestimmt. Gemessen wurde die Höhe des Basisschaums sowie der Schaumzerfall nach 1, 3 und 5 Minuten. Die Ergebnisse sind in Tab.3 zusammengefaßt.

**Tab.3:**

| Schaumvermögen Prozentangaben als Gew.-% | | | | | | | | | |
|---|---|---|---|---|---|---|---|---|---|
| Bsp. | Rezeptur* | | | | | Schaumhöhe (ml) | | | |
| | A2 % | A3% | B % | C % | D % | 0' | 1' | 3' | 5' |
| 11 | 1 | - | 37 | 2 | - | 110 | 110 | 100 | 100 |
| 12 | - | 1 | 37 | 2 | - | 120 | 110 | 100 | 100 |
| 13 | 1 | - | 37 | - | 8 | 110 | 105 | 100 | 100 |
| 14 | - | 1 | 37 | - | 8 | 110 | 105 | 100 | 100 |
| V5 | - | - | 37 | 2 | - | 110 | 100 | 90 | 90 |
| V6 | - | - | 37 | - | 8 | 110 | 110 | 100 | 90 |

| | | | | | | | | | |
|---|---|---|---|---|---|---|---|---|---|
| *) Wasser ad 100 Gew.-% | | | | | | | | | |

## Patentansprüche

1. Schäumende Detergensgemische enthaltend
a) quaternierte Fettsäurealkanolaminester der Formel (I), 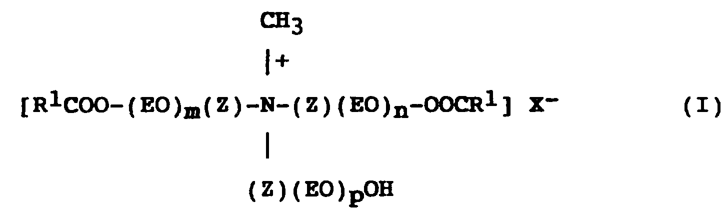 in der
R¹CO für einen aliphatischen Acylrest mit 6 bis 22 Kohlenstoffatomen und 0 oder 1 Doppelbindung,
Z für eine Ethylen- oder Isopropylengruppe,
EO für eine CH₂CH₂O-Einheit,
(m+n+p) für Zahlen von 1 bis 10 und
X für ein Halogenid, Methosulfat- oder Alkylphosphatanion steht,
und
b) weitere anionische, nichtionische und/oder amphotere bzw. zwitterionische Tenside.

2. Schäumende Detergensgemische nach Anspruch 1, **dadurch gekennzeichnet,** daß die anionischen Tenside ausgewählt sind aus der Gruppe, die von Alkylbenzolsulfonaten, Alkansulfonaten, Olefinsulfonaten, Alkylethersulfonaten, Glycerinethersulfonaten, α-Methylestersulfonaten, Sulfofettsäuren, Alkylsulfaten, Fettalkoholethersulfaten, Glycerinethersulfaten, Hydroxymischethersulfaten, Monoglycerid(ether)sulfaten, Fettsäureamid(ether)sulfaten, Sulfosuccinaten, Sulfosuccinamaten, Sulfotriglyceriden, Ethercarbonsäuren, Alkyloligoglucosidsulfaten, Alkyl(ether)phosphaten und Eiweißfettsäurekondensate gebildet wird.

3. Schäumende Detergensgemische nach Anspruch 1, **dadurch gekennzeichnet,** daß die nichtionischen Tenside ausgewählt sind aus der Gruppe, die von Fettalkoholpolyglycolethern, Alkylphenolpolyglycolethern, Fettaminpolyglycolethern, Fettsäureamidpolyglycolethern, Fettsäurepolyglycolestern, alkoxylierten Triglyceriden, Alkyloligoglykosiden, Zuckerestern, Sorbitanestern, Polysorbaten, Polyolfettsäureestern, Aminoxiden, Fettsäurealkanolamiden, Alkyllactamen und Fettsäure-N-alkylglucamiden gebildet wird.

4. Schäumende Detergensgemische nach Anspruch 1, **dadurch gekennzeichnet,** daß die amphoteren bzw. zwitterionischen Tenside ausgewählt sind aus der Gruppe, die von Alkylamidobetainen, Aminopropionaten, Aminoglycinaten, Imidazoliniumbetainen und Sulfobetainen gebildet wird.

5. Schäumende Detergensgemische nach Anspruch 1, **dadurch gekennzeichnet,** daß die quaternierten Fettsäurealkanolaminester in Mengen von 0,1 bis 90 Gew.-% - bezogen auf den Feststoffgehalt der Gemische - enthalten sind.

6. Pulverförmige Universalwaschmittel, enthaltend 10 bis 30 Gew.-% - bezogen auf das Mittel - einer Mischung von quaternierten Fettsäureestern der Formel **(I),** anionischen, nichtionischen und/oder amphoteren bzw. zwitterionischen Tensiden sowie übliche Hilfs- und Zusatzstoffe.

7. Flüssige Universalwaschmittel, enthaltend 10 bis 70 Gew.-% - bezogen auf das Mittel - einer Mischung von quaternierten Fettsäurealkanolaminestern der Formel **(I),** anionischen, nichtionischen und/oder amphoteren bzw. zwitterionischen Tensiden sowie übliche Hilfs- und Zusatzstoffe.

8. Flüssige Feinwaschmittel, enthaltend 10 bis 50 Gew.-% - bezogen auf das Mittel - einer Mischung von quaternierten Fettsäurealkanolaminestern der Formel **(I),** anionischen, nichtionischen und/oder amphoteren bzw. zwitterionischen Tensiden sowie übliche Hilfs- und Zusatzstoffe.

9. Handgeschirrspülmittel, enthaltend 10 bis 50 Gew.-% - bezogen auf das Mittel - einer Mischung von quaternierten Fettsäurealkanolaminestern der Formel **(I),** anionischen, nichtionischen und/oder amphoteren bzw. zwitterionischen Tensiden sowie übliche Hilfs- und Zusatzstoffe.

10. Flüssige Reinigungs- und Desinfektionsmittel, enthaltend 10 bis 30 Gew.-% - bezogen auf das Mittel - einer Mischung von quaternierten Fettsäurealkanolaminestern der Formel **(I),** anionischen, nichtionischen und/oder amphoteren bzw. zwitterionischen Tensiden sowie übliche Hilfs- und Zusatzstoffe.

11. Haarshampoos, enthaltend 10 bis 30 Gew.-% - bezogen auf das Mittel - einer Mischung von quaternierten Fettsäurealkanolaminestern der Formel **(I),** anionischen, nichtionischen und/oder amphoteren bzw. zwitterionischen Tensiden sowie übliche Hilfs- und Zusatzstoffe.

12. Haarspülungen, enthaltend 10 bis 30 Gew.-% - bezogen auf das Mittel - einer Mischung von quaternierten Fettsäurealkanolaminestern der Formel **(I),** anionischen, nichtionischen und/ oder amphoteren bzw. zwitterionischen Tensiden sowie übliche Hilfs- und Zusatzstoffe.

13. Schaumbäder, enthaltend 10 bis 30 Gew.-% - bezogen auf das Mittel - einer Mischung von quaternierten Fettsäurealkanolaminestern der Formel **(I),** anionischen, nichtionischen und/ oder amphoteren bzw. zwitterionischen Tensiden übliche Hilfs- und Zusatzstoffe.

14. Verwendung von Detergensgemischen nach Anspruch 1 zur Herstellung von Wasch-, Spül- und Reinigungsmitteln sowie Produkten der Haar- und Körperpflege.

## Claims

1. Foaming detergent mixtures containing
a) quaternized fatty acid alkanolamine esters corresponding to formula (I): 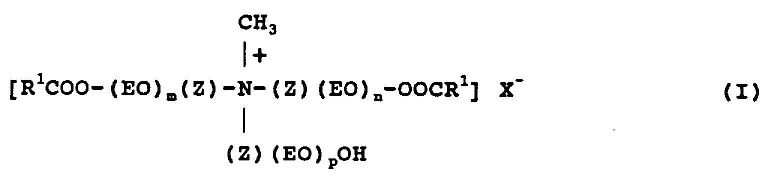 in which
R¹CO is an aliphatic acyl group containing 6 to 22 carbon atoms and 0 or 1 double bond,
Z is an ethylene or isopropylene group,
EO is a CH₂CH₂O unit,
(m+n+p) stands for numbers of 1 to 10 and
X is a halide, methosulfate or alkylphosphate anion,
and
b) other anionic, nonionic and/or amphoteric or zwitterionic surfactants.

2. Foaming detergent mixtures as claimed in claim 1, **characterized in that** the anionic surfactants are selected from the group consisting of alkylbenzenesulfonates, alkanesulfonates, olefin sulfonates, alkylether sulfonates, glycerol ether sulfonates, α-methyl ester sulfonates, sulfofatty acids, alkylsulfates, fatty alcohol ether sulfates, glycerol ether sulfates, hydroxy mixed ether sulfates, monoglyceride (ether) sulfates, fatty acid amide (ether) sulfates, sulfosuccinates, sulfosuccinamates, sulfotriglycerides, ether carboxylic acids, alkyl oligoglucoside sulfates, alkyl(ether) phosphates and protein fatty acid condensates.

3. Foaming detergent mixtures as claimed in claim 1, **characterized in that** the nonionic surfactants are selected from the group consisting of fatty alcohol polyglycol ethers, alkylphenol polyglycol ethers, fatty amine polyglycol ethers, fatty acid amide polyglycol ethers, fatty acid polyglycol esters, alkoxylated triglycerides, alkyl oligoglycosides, sugar esters, sorbitan esters, polysorbates, polyol fatty acid esters, amine oxides, fatty acid alkanolamides, alkyl lactams and fatty acid N-alkyl glucamides.

4. Foaming detergent mixtures as claimed in claim 1, **characterized in that** the amphoteric or zwitterionic surfactants are selected from the group consisting of alkylamidobetaines, aminopropionates, aminoglycinates, imidazolinium betaines and sulfobetaines.

5. Foaming detergent mixtures as claimed in claim 1, **characterized in that** the quaternized fatty acid alkanolamine esters are present in quantities of 0.1 to 90% by weight, based on the solids content of the mixtures.

6. Powder-form universal detergents containing 10 to 30% by weight, based on the detergent, of a mixture of quaternized fatty acid esters corresponding to formula (I), anionic, nonionic and/or amphoteric or zwitterionic surfactants and also typical auxiliaries and additives.

7. Liquid universal detergents containing 10 to 70% by weight, based on the detergent, of a mixture of quaternized fatty acid alkanolamine esters corresponding to formula (I), anionic, nonionic and/or amphoteric or zwitterionic surfactants and also typical auxiliaries and additives.

8. Liquid light-duty detergents containing 10 to 50% by weight, based on the detergent, of a mixture of quaternized fatty acid alkanolamine esters corresponding to formula (I), anionic, nonionic and/or amphoteric or zwitterionic surfactants and also typical auxiliaries and additives.

9. Manual dishwashing detergents containing 10 to 50% by weight, based on the detergent, of a mixture of quaternized fatty acid alkanolamine esters corresponding to formula (I), anionic, nonionic and/or amphoteric or zwitterionic surfactants and also typical auxiliaries and additives.

10. Liquid cleaners and disinfectants containing 10 to 30% by weight, based on the cleaner/disinfectant, of a mixture of quaternized fatty acid alkanolamine esters corresponding to formula (I), anionic, nonionic and/or amphoteric or zwitterionic surfactants and also typical auxiliaries and additives.

11. Hair shampoos containing 10 to 30% by weight, based on the shampoo, of a mixture of quaternized fatty acid alkanolamine esters corresponding to formula (I), anionic, nonionic and/or amphoteric or zwitterionic surfactants and also typical auxiliaries and additives.

12. Hair rinses containing 10 to 30% by weight, based on the hair rinse, of a mixture of quaternized fatty acid alkanolamine esters corresponding to formula (I), anionic, nonionic and/or amphoteric or zwitterionic surfactants and also typical auxiliaries and additives.

13. Foam baths containing 10 to 30% by weight, based on the foam bath, of a mixture of quaternized fatty acid alkanolamine esters corresponding to formula (I), anionic, nonionic and/or amphoteric or zwitterionic surfactants and also typical auxiliaries and additives.

14. The use of the detergent mixtures claimed in claim 1 for the production of laundry detergents, dishwashing detergents and cleaning products and also hair-care and personal hygiene products.

## Revendications

1. Mélanges détergents moussants contenant
a) des esters d'alcanolamine d'acides gras quaternisés de la formule (I), 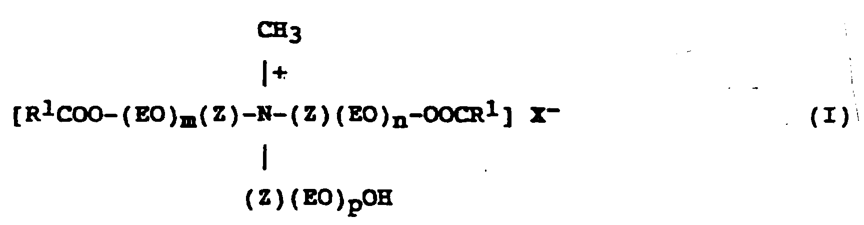 dans laquelle
R¹CO représente un radical acyle aliphatique comportant 6 à 22 atomes de carbone et 0 ou 1 double liaison,
Z est un groupe éthylène ou isopropylène,
EO représente une unité CH₂CH₂O,
(m+n+p) sont des nombres de 1 à 10 et
X représente un anion halogénure, méthosulfate ou alkylphosphate,
et
b) d'autres surfactifs anioniques, non ioniques et/ou amphotères ou zwitterioniques.

2. Mélanges détergents moussants selon la revendication 1, **caractérisés en ce que** les tensioactifs anioniques sont sélectionnés parmi le groupe constitués des alkylbenzènesulfonates, alcanesulfonates, sulfonates d'oléfine, alkyléthersulfonates, éthersulfonates de glycérine, estersulfonates d'α-méthyle, acides gras sulfoniques, alkylsulfates, éthersulfates d'alcools gras, éthersulfates de glycérine, hydroxyéthersulfates mixtes, (éther)sulfates de monoglycéride, (éther)sulfates d'amides d'acides gras, sulfosuccinates, sulfosuccinamates, sulfotriglycérides, acides éthercarboxyliques, alkyloligoglucosidesulfates, alkyl(éther)-phosphates et condensés d'acides gras protéiniques.

3. Mélanges détergents moussants selon la revendication 1, **caractérisés en ce que** les tensioactifs anioniques sont sélectionnés parmi le groupe constitués des polyglycoléthers d'alcools gras, polyglycoléthers d'alkylphénols, polyglycoléthers d'amines grasses, Polyglycoléthers d'amides d'acides gras, polyglycolesters d'acides gras, triglycérides alcoxylés, alkyl-oligoglycosides, esters de sucre, esters de sorbitane, polysorbates, esters d'acides gras de polyols, aminoxydes, alcanolamides d'acides gras, alkyllactames et N-alkylglucamides d'acides gras.

4. Mélanges détergents moussants selon la revendication 1, **caractérisés en ce que** les tensioactifs amphotères ou zwitterioniques sont sélectionnés parmi le groupe constitué des alkylamidobétaïnes, aminopropionates, aminoglycinates, imidazoliniumbétaïnes et sulfobétaïnes.

5. Mélanges détergents moussants selon la revendication 1, **caractérisés en ce** que les esters d'alcanolamine d'acides gras quaternisés sont contenus en quantités de 0,1 à 90 % en poids par rapport à la concentration en matières solides des mélanges.

6. Détergents universels en poudre contenant 10 à 30 % en poids (par rapport au produit) d'un mélange d'esters d'alcanolamine d'acides gras quaternisés de la formule (I), de tensioactifs anioniques, non ioniques et/ou amphotères ou zwitterioniques, ainsi que d'agents auxiliaires et additifs usuels.

7. Détergents universels liquides, contenant 10 à 70 % en poids (par rapport au produit) d'un mélange d'esters d'alcanolamine d'acides gras quaternisés de la formule (I), de tensioactifs anioniques, non ioniques et/ou amphotères ou zwitterioniques, ainsi que d'agents auxiliaires et additifs usuels.

8. Détergents liquides pour lingerie fine, contenant 10 à 50 % en poids (par rapport au produit) d'un mélange d'esters d'alcanolamine d'acides gras quaternisés de la formule (I), de tensioactifs anioniques, non ioniques et/ou amphotères ou zwitterioniques, ainsi que d'agents auxiliaires et additifs usuels.

9. Produit pour le lavage de la vaisselle à la main, contenant 10 à 50 % en poids (par rapport au produit) d'un mélange d'esters d'alcanolamine d'acides gras quaternisés de la formule (I), de tensioactifs anioniques, non ioniques et/ou amphotères ou zwitterioniques, ainsi que d'agents auxiliaires et additifs usuels.

10. Produits de nettoyage et de désinfection liquides, contenant 10 à 30 % en poids (par rapport au produit) d'un mélange d'esters d'alcanolamine d'acides gras quaternisés de la formule (I), de tensioactifs anioniques, non ioniques et/ou amphotères ou zwitterioniques, ainsi que d'agents auxiliaires et additifs usuels.

11. Shampooings pour les cheveux, contenant 10 à 30 % en poids (par rapport au produit) d'un mélange d'esters d'alcanolamine d'acides gras quaternisés de la formule (I), de tensioactifs anioniques, non ioniques et/ou amphotères ou zwitterioniques, ainsi que d'agents auxiliaires et additifs usuels.

12. Rinçages pour les cheveux, contenant 10 à 30 % en poids (par rapport au produit) d'un mélange d'esters d'alcanolamine d'acides gras quaternisés de la formule (I), de tensioactifs anioniques, non ioniques et/ou amphotères ou zwitterioniques, ainsi que d'agents auxiliaires et additifs usuels.

13. Bains moussants, contenant 10 à 30 % en poids (par rapport au produit) d'un mélange d'esters d'alcanolamine d'acides gras quaternisés de la formule (I), de tensioactifs anioniques, non ioniques et/ou amphotères ou zwitterioniques, ainsi que d'agents auxiliaires et additifs usuels.

14. Utilisation des mélanges détergents selon la revendication 1 pour la fabrication de produits de lavage, de rinçage et de nettoyage ainsi que de produits pour le soin de la chevelure et du corps.
